# EUROPEAN PATENT APPLICATION

(11) **EP 4 461 727 A1**
(43) Date of publication of application: **13.11.2024**
(21) Application number: 23746087.8
(22) Date of filing: 16.01.2023
(51) Int. Cl.: C07D 213/72, C07D 213/60, A61K 31/435, A61K 31/44, A61P 9/04, A61P 9/10

(54) **CRYSTAL FORM OF 7-AZASPIRO[4,5]DECANE-6,10-DIONE COMPOUND AND PREPARATION METHOD THEREFOR**

(30) Priority: 27.01.2022 CN 202210103134; 23.08.2022 CN 202211017556
(71) Applicant: Medshine Discovery Inc., Nanjing, Jiangsu 210032 (CN); Soter Biopharma Pte. Ltd., Singapore 179803 (SG)
(72) Inventor: YAN, Xiaobing, Shanghai 200131 (CN); LAI, Wei, Shanghai 200131 (CN); QIAN, Wenyuan, Shanghai 200131 (CN)
(74) Representative: Cabinet Becker et Associés
(86) International application number: PCT/CN2023/072433
(87) International publication number: WO 2023/143205

(57) **Abstract**

Disclosed in the present invention are a crystal form of a class of 7-azaspiro[4,5]decane-6,10-dione compounds and a preparation method therefor. Specifically disclosed are a method for preparing a compound of formula (I) and a crystal form thereof and the use of the compound and the crystal form thereof.

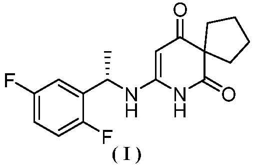

## Description

**This application claims the priority of:**
CN202210103134.0, filed on January 27, 2022;
CN202211017556.2, filed on August 23, 2022.

### TECHNICAL FIELD

The present application discloses a crystal form of a class of 7-azaspiro[4,5]decane-6,10-dione compounds and a preparation method thereof, specifically discloses a preparation method and use of a compound of formula (I) and a crystal form thereof.

### BACKGROUND

Hypertrophic cardiomyopathy (HCM) is a myocardial disease characterized by myocardial hypertrophy, which often invades the interventricular septum, causing the ventricular cavity to become smaller, obstructing the blood filling of left ventricular, and decreasing the diastolic compliance of left ventricular. Hypertrophic cardiomyopathy is divided into obstructive and non-obstructive hypertrophic cardiomyopathy depending on whether there is obstruction in the left ventricular outflow tract, which may be related to genetics, etc. The global incidence of HCM is about 1/500, and the clinical manifestations of HCM are diverse. It may be asymptomatic, or it may cause palpitations, dyspnea on exertion, dull pain in the precordial area, fatigue, syncope and even sudden death. In the late stage, symptoms of left-sided heart failure appear.

Currently, there are limited drugs for the treatment of HCM. The treatment of HCM is mainly to improve symptoms through beta-blockers or calcium channel blockers, which cannot target the cause, delay the progression of myocardial hypertrophy, or improve prognosis. The treatment effect is limited.

Myosin and actin are the material basis of myocardial contraction. Cross-bridges of myosin periodically bind to and dissociate from actin, driving the sliding of myofilaments and leading to myocardial contraction. Myosin has ATPase activity and provides power for the myocardial contraction by hydrolyzing ATP. Myosin mutations may lead to a prolonged binding time between myosin and actin, excessive contraction and impaired relaxation of the left ventricular myocardium, leading to left ventricular hypertrophy and fibrosis, and inducing HCM. MYK-461 is an allosteric regulator of cardiac myosin, which slows down the rate of phosphohydrolysis and reduces the binding time of myosin and actin, producing a negative inotropic effect and alleviating pathological changes such as myocardial hypertrophy caused by excessive contraction of the left ventricular myocardium. However, the drug is eliminated slowly from the body and stays in the body for too long, making it difficult to adjust the dosage quickly. Therefore, the development of myosin inhibitors with better activity and more ideal pharmacokinetic properties has important clinical value and significance.

In addition, abnormalities in cardiac sarcomeres have been identified as the driving cause of a variety of cardiac diseases and conditions, such as diastolic heart failure with preserved ejection fraction, ischemic heart disease, angina pectoris, and restrictive cardiomyopathy, etc. Myosin ATPase inhibitors may also have a potential therapeutic effect in alleviating the pathological process of the above diseases by inhibiting myocardial contraction.

### SUMMARY

The present disclosure provides a crystal form A of a compound of formula (I), which is characterized by X-ray powder diffraction pattern (XRPD) with characteristic diffraction peaks at 2θ angles of 18.283±0.200°, 19.662±0.200°, and 22.420±0.200°;

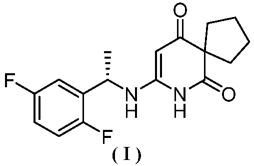

In some embodiments of the present disclosure, the above crystal form A has X-ray powder diffraction pattern with characteristic diffraction peaks at 2θ angles of 11.143±0.200°, 18.283±0.200°, 19.662±0.200°, 22.420±0.200°, 26.259±0.200°, and 28.056±0.200°.

In some embodiments of the present disclosure, the above crystal form A has X-ray powder diffraction pattern with characteristic diffraction peaks at 2θ angles of 11.143±0.200°, 18.283±0.200°, 19.662±0.200°, 22.420±0.200°, 26.259±0.200°, 27.261±0.200°, 28.056±0.200°, and 30.256±0.200°.

In some embodiments of the present disclosure, disclosed is the above crystal form A, which is characterized by X-ray powder diffraction pattern comprising at least 5, 6, 7 or 8 characteristic diffraction peaks represented by 2θ angles selected from the following: 11.143±0.200°, 18.283±0.200°, 19.662±0.200°, 22.420±0.200°, 26.259±0.200°, 27.261±0.200°, 28.056±0.200°, and 30.256±0.200°.

In some embodiments of the present disclosure, the above crystal form A has X-ray powder diffraction pattern with characteristic diffraction peaks at 2θ angles of 11.143±0.200°, 16.522±0.200°, 17.053±0.200°, 18.283±0.200°, 19.662±0.200°, 22.420±0.200°, 23.909±0.200°, 26.259±0.200°, 27.261±0.200°, 28.056±0.200°, 30.256±0.200°, and 33.761±0.200°.

In some embodiments of the present disclosure, disclosed is the above crystal form A, which is characterized by X-ray powder diffraction pattern comprising at least 5, 6, 7, 8, 9, 10, 11 or 12 characteristic diffraction peaks represented by 2θ angles selected from the following: 11.143±0.200°, 16.522±0.200°, 17.053±0.200°, 18.283±0.200°, 19.662±0.200°, 22.420±0.200°, 23.909±0.200°, 26.259±0.200°, 27.261±0.200°, 28.056±0.200°, 30.256±0.200°, and 33.761±0.200°.

In some embodiments of the present disclosure, the above crystal form A has X-ray powder diffraction pattern with characteristic diffraction peaks at 2θ angles of 11.143±0.200°, 13.260±0.200°, 16.522±0.200°, 17.053±0.200°, 18.283±0.200°, 19.662±0.200°, 22.420±0.200°, 23.568±0.200°, 23.909±0.200°, 26.259±0.200°, 27.261±0.200°, 28.056±0.200°, 29.008±0.200°, 30.256±0.200°, 33.761±0.200°, and 35.404±0.200°.

In some embodiments of the present disclosure, the above crystal form A has X-ray powder diffraction pattern with characteristic diffraction peaks at 2θ angles of 11.143±0.200°, 13.260±0.200°, 14.860±0.200°, 16.163±0.200°, 16.522±0.200°, 17.053±0.200°, 17.537±0.200°, 18.283±0.200°, 19.662±0.200°, 22.420±0.200°, 23.568±0.200°, 23.909±0.200°, 26.259±0.200°, 26.726±0.200°, 27.261±0.200°, 28.056±0.200°, 29.008±0.200°, 30.256±0.200°, 31.219±0.200°, 31.646±0.200°, 32.037±0.200°, 32.438±0.200°, 32.807±0.200°, 33.761±0.200°, 34.534±0.200°, 35.404±0.200°, 36.856±0.200°, 37.813±0.200°, and 39.456±0.200°.

In some embodiments of the present disclosure, the above crystal form A has X-ray powder diffraction pattern with characteristic diffraction peaks at 2θ angles of 11.143±0.100°, 13.260±0.100°, 14.860±0.100°, 16.163±0.100°, 16.522±0.100°, 17.053±0.100°, 17.537±0.100°, 18.283±0.100°, 19.662±0.100°, 22.420±0.100°, 23.568±0.100°, 23.909±0.100°, 26.259±0.100°, 26.726±0.100°, 27.261±0.100°, 28.056±0.100°, 29.008±0.100°, 30.256±0.100°, 31.219±0.100°, 31.646±0.100°, 32.037±0.100°, 32.438±0.100°, 32.807±0.100°, 33.761±0.100°, 34.534±0.100°, 35.404±0.100°, 36.856±0.100°, 37.813±0.100°, and 39.456±0.100°.

In some embodiments of the present disclosure, the above crystal form A has X-ray powder diffraction pattern with characteristic diffraction peaks at 2θ angles of 11.143°, 13.260°, 14.860°, 16.163°, 16.522°, 17.053°, 17.537°, 18.283°, 19.662°, 22.420°, 23.568°, 23.909°, 26.259°, 26.726°, 27.261°, 28.056°, 29.008°, 30.256°, 31.219°, 31.646°, 32.037°, 32.438°, 32.807°, 33.761°, 34.534°, 35.404°, 36.856°, 37.813°, and 39.456°.

In some embodiments of the present disclosure, the above crystal form A has X-ray powder diffraction pattern with characteristic diffraction peaks at 2θ angles of 18.283±0.200°, 19.662±0.200°, 22.420±0.200°, and/or 11.143±0.200°, and/or 13.260±0.200°, and/or 14.860±0.200°, and/or 16.163±0.200°, and/or 16.522±0.200°, and/or 17.053±0.200°, and/or 17.537±0.200°, and/or 23.568±0.200°, and/or 23.909±0.200°, and/or 26.259±0.200°, and/or 26.726±0.200°, and/or 27.261±0.200°, and/or 28.056±0.200°, and/or 29.008±0.200°, and/or 30.256±0.200°, and/or 31.219±0.200°, and/or 31.646±0.200°, and/or 32.037±0.200°, and/or 32.438±0.200°, and/or 32.807±0.200°, and/or 33.761±0.200°, and/or 34.534±0.200°, and/or 35.404±0.200°, and/or 36.856±0.200°, and/or 37.813±0.200°, and/or 39.456±0.200°.

In some embodiments of the present disclosure, the above crystal form A has X-ray powder diffraction pattern with characteristic diffraction peaks at 2θ angles of 18.283±0.100°, 19.662±0.100°, 22.420±0.100°, and/or 11.143±0.100°, and/or 13.260±0.100°, and/or 14.860±0.100°, and/or 16.163±0.100°, and/or 16.522±0.100°, and/or 17.053±0.100°, and/or 17.537±0.100°, and/or 23.568±0.100°, and/or 23.909±0.100°, and/or 26.259±0.100°, and/or 26.726±0.100°, and/or 27.261±0.100°, and/or 28.056±0.100°, and/or 29.008±0.100°, and/or 30.256±0.100°, and/or 31.219±0.100°, and/or 31.646±0.100°, and/or 32.037±0.100°, and/or 32.438±0.100°, and/or 32.807±0.100°, and/or 33.761±0.100°, and/or 34.534±0.100°, and/or 35.404±0.100°, and/or 36.856±0.100°, and/or 37.813±0.100°, and/or 39.456±0.100°.

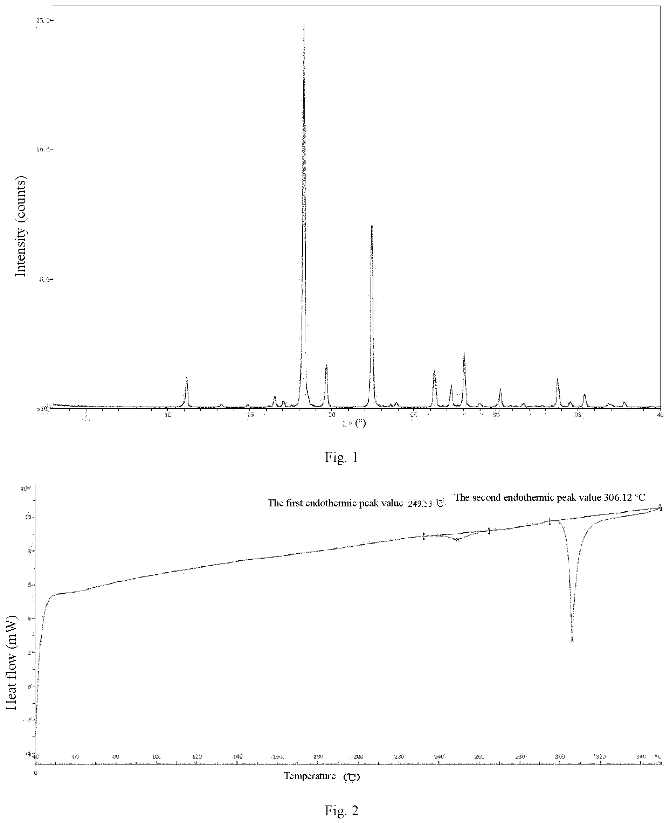

In some embodiments of the present disclosure, the XRPD pattern of the above crystal form A is substantially as shown in Fig. 1.

In some embodiments of the present disclosure, the XRPD pattern resolution data of the above crystal form A are shown in Table 1.

**Table 1 XRPD pattern resolution data of the crystal form A of the compound of formula (I)**

| No. | 2θ[°] | Peak height [cts] | D-spacing [Å] | Relative intensity [%] | No. | 2θ[°] | Peak height [cts] | D-spacing [Å] | Relative intensity [%] |
|---|---|---|---|---|---|---|---|---|---|
| 1 | 11.143 | 1135 | 7.9341 | 7.8 | 16 | 28.056 | 2129 | 3.1778 | 14.9 |
| 2 | 13.260 | 153 | 6.6716 | 1.0 | 17 | 29.008 | 154 | 3.0757 | 1.2 |
| 3 | 14.860 | 102 | 5.9567 | 0.6 | 18 | 30.256 | 700 | 2.9516 | 5.0 |
| 4 | 16.163 | 58 | 5.4792 | 0.7 | 19 | 31.219 | 44 | 2.8627 | 0.5 |
| 5 | 16.522 | 395 | 5.3612 | 3.9 | 20 | 31.646 | 139 | 2.8251 | 0.9 |
| 6 | 17.053 | 261 | 5.1953 | 2.1 | 21 | 32.037 | 40 | 2.7915 | 0.2 |
| 7 | 17.537 | 77 | 5.0531 | 0.6 | 22 | 32.438 | 44 | 2.7579 | 0.5 |
| 8 | 18.283 | 14765 | 4.8486 | 100.0 | 23 | 32.807 | 48 | 2.7277 | 0.5 |
| 9 | 19.662 | 1641 | 4.5113 | 11.4 | 24 | 33.761 | 1081 | 2.6527 | 8.0 |
| 10 | 22.420 | 7011 | 3.9624 | 43.6 | 25 | 34.534 | 184 | 2.5951 | 1.4 |
| 11 | 23.568 | 113 | 3.7719 | 0.9 | 26 | 35.404 | 503 | 2.5333 | 3.6 |
| 12 | 23.909 | 204 | 3.7188 | 1.9 | 27 | 36.856 | 132 | 2.4368 | 1.5 |
| 13 | 26.259 | 1489 | 3.3911 | 11.3 | 28 | 37.813 | 183 | 2.3773 | 1.4 |
| 14 | 26.726 | 56 | 3.3329 | 0.7 | 29 | 39.456 | 40 | 2.2820 | 0.3 |
| 15 | 27.261 | 888 | 3.2687 | 6.4 | | | | | |

In some embodiments of the present disclosure, disclosed is the above crystal form A, which has a differential scanning calorimetry curve with peak values of the endothermic peaks at 249.53±8 °C and 306.12±8 °C.

In some embodiments of the present disclosure, disclosed is the above crystal form A, which has a differential scanning calorimetry curve with peak values of the endothermic peaks at 249.53±3 °C and 306.12±3 °C.

In some embodiments of the present disclosure, disclosed is the above crystal form A, which has a DSC curve substantially as shown in Fig. 2.

In some embodiments of the present disclosure, disclosed is the above crystal form A, which has a thermogravimetric analysis curve with a weight loss of up to 0.075% at 200±3 °C, and a weight loss of up to 0.164% at 250±3 °C.

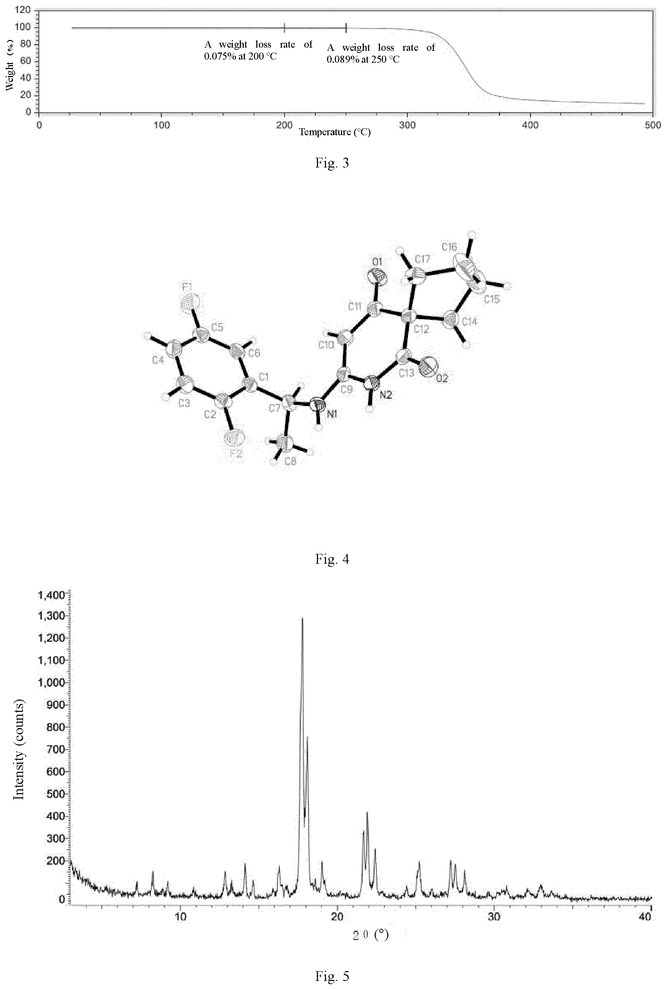

In some embodiments of the present disclosure, disclosed is the above crystal form A, which has a TGA curve substantially as shown in Fig. 3.

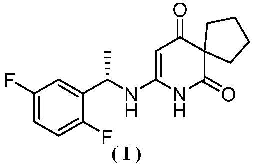

In some embodiments of the present disclosure, the above crystal form B has X-ray powder diffraction pattern with characteristic diffraction peaks at 2θ angles of 12.861±0.200°, 14.135±0.200°, 17.788±0.200°, 18.099±0.200°, 21.659±0.200°, 21.912±0.200°, 22.410±0.200°, and 25.218±0.200°.

In some embodiments of the present disclosure, disclosed is the above crystal form B, which is characterized by X-ray powder diffraction pattern comprising at least 5, 6, 7 or 8 characteristic diffraction peaks represented by 2Θ angles selected from the following: 12.861±0.200°, 14.135±0.200°, 17.788±0.200°, 18.099±0.200°, 21.659±0.200°, 21.912±0.200°, 22.410±0.200°, and 25.218±0.200°.

In some embodiments of the present disclosure, the above crystal form B has X-ray powder diffraction pattern with characteristic diffraction peaks at 2θ angles of 8.262±0.200°, 12.861±0.200°, 14.135±0.200°, 16.303±0.200°, 17.788±0.200°, 18.099±0.200°, 19.053±0.200°, 21.659±0.200°, 21.912±0.200°, 22.410±0.200°, 25.218±0.200°, 27.230±0.200°, 27.526±0.200°, and 28.132±0.200°.

In some embodiments of the present disclosure, disclosed is the above crystal form B, which is characterized by X-ray powder diffraction pattern comprising at least 5, 6, 7, 8, 9, 10, 11, 12, 13 or 14 characteristic diffraction peaks represented by 2θ angles selected from the following: 8.262±0.200°, 12.861±0.200°, 14.135±0.200°, 16.303±0.200°, 17.788±0.200°, 18.099±0.200°, 19.053±0.200°, 21.659±0.200°, 21.912±0.200°, 22.410±0.200°, 25.218±0.200°, 27.230±0.200°, 27.526±0.200°, and 28.132±0.200°.

In some embodiments of the present disclosure, the above crystal form B has X-ray powder diffraction pattern with characteristic diffraction peaks at 2θ angles of 7.240±0.200°, 8.262±0.200°, 9.189±0.200°, 10.841±0.200°, 12.861±0.200°, 13.269±0.200°, 14.135±0.200°, 14.651±0.200°, 15.907±0.200°, 16.303±0.200°, 16.770±0.200°, 17.788±0.200°, 18.099±0.200°, 19.053±0.200°, 21.659±0.200°, 21.912±0.200°, 22.410±0.200°, 22.829±0.200°, 24.408±0.200°, 25.218±0.200°, 26.009±0.200°, 27.230±0.200°, 27.526±0.200°, 28.132±0.200°, 29.630±0.200°, 32.150±0.200°, 32.961±0.200°, and 33.645±0.200°.

In some embodiments of the present disclosure, the above crystal form B has X-ray powder diffraction pattern with characteristic diffraction peaks at 2θ angles of 7.240±0.100°, 8.262±0.100°, 9.189±0.100°, 10.841±0.100°, 12.861±0.100°, 13.269±0.100°, 14.135±0.100°, 14.651±0.100°, 15.907±0.100°, 16.303±0.100°, 16.770±0.100°, 17.788±0.100°, 18.099±0.100°, 19.053±0.100°, 21.659±0.100°, 21.912±0.100°, 22.410±0.100°, 22.829±0.100°, 24.408±0.100°, 25.218±0.100°, 26.009±0.100°, 27.230±0.100°, 27.526±0.100°, 28.132±0.100°, 29.630±0.100°, 32.150±0.100°, 32.961±0.100°, and 33.645±0.100°.

In some embodiments of the present disclosure, the above crystal form B has X-ray powder diffraction pattern with characteristic diffraction peaks at 2θ angles of 7.240°, 8.262°, 9.189°, 10.841°, 12.861°, 13.269°, 14.135°, 14.651°, 15.907°, 16.303°, 16.770°, 17.788°, 18.099°, 19.053°, 21.659°, 21.912°, 22.410°, 22.829°, 24.408°, 25.218°, 26.009°, 27.230°, 27.526°, 28.132°, 29.630°, 32.150°, 32.961°, and 33.645°.

In some embodiments of the present disclosure, the above crystal form B has X-ray powder diffraction pattern with characteristic diffraction peaks at 2θ angles of 17.788°±0.200°, 18.099±0.200°, 21.659±0.200°, and/or 21.912±0.200°, and/or 7.240±0.200°, and/or 8.262±0.200°, and/or 9.189±0.200°, and/or 10.841±0.200°, and/or 12.861±0.200°, and/or 13.269±0.200°, and/or 14.135±0.200°, and/or 14.651±0.200°, and/or 15.907±0.200°, and/or 16.303±0.200°, and/or 16.770±0.200°, and/or 19.053±0.200°, and/or 22.410±0.200°, and/or 22.829±0.200°, and/or 24.408±0.200°, and/or 25.218±0.200°, and/or 26.009±0.200°, and/or 27.230±0.200°, and/or 27.526±0.200°, and/or 28.132±0.200°, and/or 29.630±0.200°, and/or 32.150±0.200°, and/or 32.961±0.200°, and/or 33.645±0.200°.

In some embodiments of the present disclosure, the above crystal form B has X-ray powder diffraction pattern with characteristic diffraction peaks at 2θ angles of 17.788°±0.100°, 18.099±0.100°, 21.659±0.100°, and/or 21.912±0.100°, and/or 7.240±0.100°, and/or 8.262±0.100°, and/or 9.189±0.100°, and/or 10.841±0.100°, and/or 12.861±0.100°, and/or 13.269±0.100°, and/or 14.135±0.100°, and/or 14.651±0.100°, and/or 15.907±0.100°, and/or 16.303±0.100°, and/or 16.770±0.100°, and/or 19.053±0.100°, and/or 22.410±0.100°, and/or 22.829±0.100°, and/or 24.408±0.100°, and/or 25.218±0.100°, and/or 26.009±0.100°, and/or 27.230±0.100°, and/or 27.526±0.100°, and/or 28.132±0.100°, and/or 29.630±0.100°, and/or 32.150±0.100°, and/or 32.961±0.100°, and/or 33.645±0.100°.

In some embodiments of the present disclosure, the XRPD pattern resolution data of the above crystal form B are shown in Table 2.

**Table 2 XRPD pattern resolution data of the crystal form B of the compound of formula (I)**

| No. | 2θ[°] | Peak height [cts] | D-spacing [Å] | Relative intensity [%] | No. | 2θ[°] | Peak height [cts] | D-spacing [Å] | Relative intensity [%] |
|---|---|---|---|---|---|---|---|---|---|
| 1 | 7.240 | 95.3229 | 12.19978 | 3.9 | 15 | 21.659 | 323.373 | 4.09986 | 22.7 |
| 2 | 8.262 | 154.957 | 10.69278 | 8.6 | 16 | 21.912 | 417.795 | 4.05312 | 30.3 |
| 3 | 9.189 | 104.853 | 9.61591 | 5.0 | 17 | 22.410 | 251.950 | 3.96412 | 16.9 |
| 4 | 10.841 | 68.8444 | 8.15463 | 2.6 | 18 | 22.829 | 63.4991 | 3.89226 | 1.9 |
| 5 | 12.861 | 151.604 | 6.87764 | 9.0 | 19 | 24.408 | 88.2752 | 3.64398 | 4.0 |
| 6 | 13.269 | 103.027 | 6.66729 | 5.1 | 20 | 25.218 | 196.697 | 3.52863 | 12.4 |
| 7 | 14.135 | 179.287 | 6.26047 | 11.4 | 21 | 26.009 | 70.0000 | 3.42313 | 2.3 |
| 8 | 14.651 | 106.116 | 6.04121 | 5.6 | 22 | 27.230 | 193.477 | 3.27241 | 12.2 |
| 9 | 15.907 | 58.9828 | 5.56714 | 1.5 | 23 | 27.526 | 183.975 | 3.23783 | 11.4 |
| 10 | 16.303 | 163.852 | 5.43249 | 9.8 | 24 | 28.132 | 156.746 | 3.16940 | 9.4 |
| 11 | 16.770 | 82.3403 | 5.28244 | 3.2 | 25 | 29.630 | 55.6683 | 3.01253 | 1.8 |
| 12 | 17.788 | 1288.82 | 4.98221 | 100.0 | 26 | 32.150 | 64.4326 | 2.78196 | 2.1 |
| 13 | 18.099 | 740.754 | 4.89732 | 55.7 | 27 | 32.961 | 92.0000 | 2.71528 | 4.3 |
| 14 | 19.053 | 188.143 | 4.65418 | 11.2 | 28 | 33.645 | 64.5000 | 2.66167 | 2.2 |

In some embodiments of the present disclosure, disclosed is the above crystal form B, which has a differential scanning calorimetry curve with a peak value of an endothermic peak at 249.48±8 °C.

In some embodiments of the present disclosure, disclosed is the above crystal form B, which has a differential scanning calorimetry curve with a peak value of an endothermic peak at 249.48±3 °C.

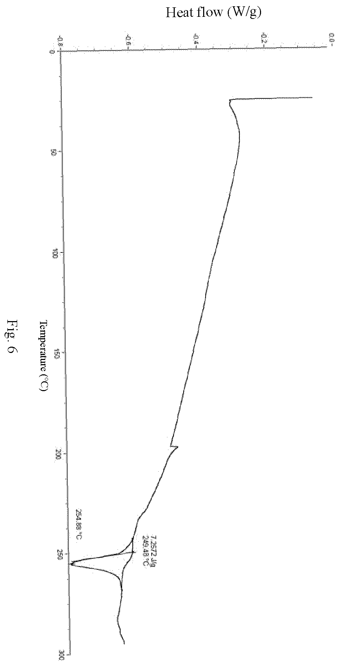

In some embodiments of the present disclosure, disclosed is the above crystal form B, which has a DSC curve substantially as shown in Fig. 6.

The present disclosure also provides a method of preparing the crystal form A of the compound of formula (I), comprising:
1) adding the compound of formula (I) into an alcohol solvent, ethyl acetate, tert-butyl methyl ether, tetrahydrofuran, or dichloromethane;
2) stirring at 15 to 100 °C for 1 to 168 hours;
3) filtering, collecting the filter cake, and vacuum drying the filter cake at 30 to 45 °C for 0.5 to 2 hours, alternatively vacuum drying the filter cake at 35 °C for 1 hour; wherein the compound of formula (I) is

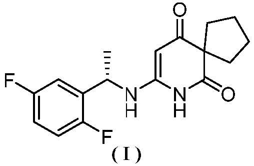

In some embodiments of the present disclosure, the above alcohol solvent is selected from methanol and ethanol.

The present disclosure also provides use of the above compound of formula (I), the above crystal form A of the compound of formula (I), the above crystal form B of the compound of formula (I), or the method of preparing the crystal form A of the compound of formula (I) in the manufacture of a medicament for treating a LSD1-related disease.

The present disclosure also provides use of the above compound of formula (I), the above crystal form A of the compound of formula (I), or the method of preparing the crystal form A of the compound of formula (I) in the manufacture of a medicament for treating a LSD1-related disease.

The present disclosure also provides a pharmaceutical composition, comprising a therapeutically effective amount of the above compound of formula (I), the above crystal form A of the compound of formula (I), or the above crystal form B of the compound of formula (I), and a pharmaceutically acceptable carrier.

The present disclosure also provides a pharmaceutical composition, comprising a therapeutically effective amount of the above compound of formula (I) or the above crystal form A of the compound of formula (I), and a pharmaceutically acceptable carrier.

The present disclosure also provides use of the above compound of formula (I), the above crystal form A of the compound of formula (I), the above crystal form B of the compound of formula (I), or the above pharmaceutical composition in the manufacture of a medicament as a cardiac myosin inhibitor.

The present disclosure also provides use of the above compound of formula (I), the above crystal form A of the compound of formula (I), or the above pharmaceutical composition in the manufacture of a medicament as a cardiac myosin inhibitor.

The present disclosure also provides use of the above compound of formula (I), the above crystal form A of the compound of formula (I), the above crystal form B of the compound of formula (I), or the above pharmaceutical composition in the manufacture of a medicament for treating heart failure and hypertrophic cardiomyopathy.

The present disclosure also provides use of the above compound of formula (I), the above crystal form A of the compound of formula (I), or the above pharmaceutical composition in the manufacture of a medicament for treating heart failure and hypertrophic cardiomyopathy.

The present disclosure also provides a method of treating a disease associated with cardiac myosin inhibitor in a subject in need thereof, comprising providing the subject with an effective dose of the compound of formula (I), the crystal form A of the compound of formula (I), the crystal form B of the compound of formula (I), or the pharmaceutical composition as defined in any of the above technical solutions.

The present disclosure also provides a method of treating a disease associated with cardiac myosin inhibitor in a subject in need thereof, comprising providing the subject with an effective dose of the compound of formula (I), the crystal form A of the compound of formula (I), or the pharmaceutical composition as defined in any of the above technical solutions.

The present disclosure also provides a method of treating heart failure and hypertrophic cardiomyopathy in a subject in need thereof, comprising providing the subject with an effective dose of the compound of formula (I), the crystal form A of the compound of formula (I), the crystal form B of the compound of formula (I), or the pharmaceutical composition as defined in any of the above technical solutions.

The present disclosure also provides a method of treating heart failure and hypertrophic cardiomyopathy in a subject in need thereof, comprising providing the subject with an effective dose of the compound of formula (I), the crystal form A of the compound of formula (I), or the pharmaceutical composition as defined in any of the above technical solutions.

### Technical effect

The crystal form A of the compound of formula (I) of the present disclosure is easy to obtain, and has good physical stability and chemical stability, as well as high industrial application value and economic value. The compound of the present disclosure has a good inhibitory effect on cardiac myosin ATPase, and has excellent pharmacokinetic properties.

### Definition and description

Unless otherwise specified, the following terms and phrases used herein are intended to have the following meanings. A specific phrase or term should not be considered indefinite or unclear in the absence of a particular definition, but should be understood in the conventional sense. When a trade name appears herein, it is intended to refer to its corresponding commodity or active ingredient thereof.

It must be noted that, unless otherwise clearly indicated by the context or clearly contradicted by this disclosure, the singular forms "a", "an", "the" and similar terms in the content of the present disclosure (especially in the content of the appended claims) as used herein and in the appended claims should be interpreted as including both the singular and plural forms. Thus, for example, reference to "the compound" includes reference to one or more compounds; etc.

The term "amorphous" or "amorphous form" is intended to mean that the substance, component or product in question lacks a characteristic crystal shape or crystalline structure, and is substantially not a crystal when determined, for example, by XRPD (X-ray powder diffraction), or the substance, component or product in question is not birefringent or cubic, when being viewed, for example, using a polarized light microscope, or does not have a sharp peak in X-ray powder diffraction pattern. In certain embodiments, a sample comprising an amorphous form of a substance may be substantially free of other amorphous forms and/or crystalline forms.

The differential scanning calorimetry (DSC) of the crystal form of the present disclosure has experimental errors, and is slightly affected by the degree of dryness of the sample. The position and peak value of an endothermic peak may be slightly different between one machine and another, and between one sample and another. The experimental error or the difference in value may be less than or equal to 10 °C, or less than or equal to 9 °C, or less than or equal to 8 °C, or less than or equal to 7 °C, or less than or equal to 6 °C, or less than or equal to 5 °C, or less than or equal to 4 °C, or less than or equal to 3 °C, or less than or equal to 2 °C, or less than or equal to 1 °C. Therefore, the peak position or peak value of a DSC endothermic peak cannot be regarded as absolute.

The intermediate compounds of the present disclosure can be prepared by a variety of synthetic methods well known to those skilled in the art, including the specific embodiments listed below, the embodiments formed by combining the specific embodiments listed below with other chemical synthetic methods, and the equivalent alternative methods well known to those skilled in the art. The alternative embodiments include, but are not limited to, the examples of the present disclosure.

Unless otherwise specified, when a double bond structure, such as a carbon-carbon double bond, a carbon-nitrogen double bond and a nitrogen-nitrogen double bond, exists in a compound, and each atom on the double bond is connected to two different substituents (in a double bond involving a nitrogen atom, a lone pair of electrons on the nitrogen atom is considered as a substituent attached to it), if the atom on the double bond and the substituents thereof in the compound are represented by 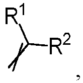 it represents the (Z) isomer or (E) isomer of the compound or a mixture of the two isomers.

The structure of the compound disclosed herein can be confirmed by conventional methods well known to those skilled in the art. If the present disclosure relates to an absolute configuration of a compound, the absolute configuration can be confirmed by conventional techniques in the art, such as single-crystal X-Ray diffraction (SXRD). In the single-crystal X-Ray diffraction (SXRD), the diffraction intensity data of the cultivated single crystal is collected using a Bruker D8 venture diffractometer with a light source of CuKα radiation in a scanning mode of ϕ/ω scan; after collecting the relevant data, the crystal structure is further analyzed by the direct method (Shelxs97) to confirm the absolute configuration.

The chemical reactions in the specific embodiments of the present disclosure are completed in a suitable solvent, which must be suitable for the chemical changes of the present disclosure and the reagents and materials required. In order to obtain the compound of the present disclosure, it is sometimes necessary for those skilled in the art to modify or select synthetic steps or reaction schemes based on the existing embodiments.

The present disclosure will be described in detail below through examples, which are not intended to limit the present disclosure in any way.

All solvents used in the present disclosure are commercially available and can be used without further purification.

The solvents used in the present disclosure are commercially available.

The following abbreviations are used in the present disclosure: TEA represents triethylamine; DIEA represents N,N-diisopropylethylamine; PE represents petroleum ether; EtOAc represents ethyl acetate; EA represents ethyl acetate; THF represents tetrahydrofuran; MeOH represents methanol; MTBE represents methyl tert-butyl ether; DCM represents dichloromethane; EtOH represents ethanol; iPrOH represents isopropyl alcohol; Boc₂O represents di-tert-butyl dicarbonate; L-selectride represents lithium tri-sec-butylborohydride; TCFH represents N,N,N',N'-tetramethylchloroformamidinium hexafluorophosphate; FA represents formic acid; TFA represents trifluoroacetic acid; ACN represents acetonitrile; TLC represents thin layer chromatography; HPLC represents high-performance liquid chromatography; LCMS represents liquid chromatography-mass spectrometry; DMSO represents dimethyl sulfoxide; DMF represents N,N-dimethylformamide; LDA represents lithium diisopropylamide; DMAC represents N,N-dimethylacetamide; PEG-400 represents polyethylene glycol 400; EGTA represents ethylene glycol-bis(2-aminoethyl ether)-N,N,N',N'-tetraacetic acid; DMSO-*d₆* represents deuterated dimethyl sulfoxide; CDCl₃ represents deuterated chloroform; BID represents twice daily; QD represents once daily; PO represents oral administration; IV represents intravenous administration.

Compounds are named manually or using ChemDraw^{®} software, and commercially available compounds are named with their vendor directory names.

### Instrument and analysis method of the present disclosure

**X-ray powder diffraction (XRPD) method one of the present disclosure, with the test parameters of the testing of crystal form A shown in Table 3.**

**Table 3 Test parameters of XRPD**

| Parameter | Set value |
|---|---|
| Model | DX-2700BH |
| X-ray | Cu, Kα (1.54184Å) |
| X-ray tube settings | 40 kV, 30 mA |
| Divergence slit | 1 mm |
| Receiving slit | 0.3 mm |
| Anti-scatter slit | 1 mm |
| Range of scanning (°2θ) | 3-40 |
| Step-width angle (°2θ) | 0.02 |
| Measuring time | 0.5 s |

**X-ray powder diffraction (XRPD) method two of the present disclosure, in which the data were acquired by D2 Phaser of Bruker instruments. The test parameters are shown in Table 4.**

**Table 4 Test parameters of XRPD**

| Name of the method | 3-40+0.02+0.2+15.bsml | Sensing mode | Step measurement |
|---|---|---|---|
| X-ray emitter | Cu, kα (λ=1.54184Å) | Voltage of the light tube (kV) | 30 |
| Current of the light tube (mA) | 10 | Divergence slit (mm) | 0.6 |
| Axial soller slit of main beam path (°) | 2.5 | Axial softer slit of secondary beam path (°) | 2.5 |
| Detector slit (°) | 0.075 | Anti-scatter slit (mm) | 1 |
| Scan axis | θs-θd | Step size (deg) | 0.02 |
| Dwell time for each step (S) | 0.2 | Range of scanning (deg) | 3-40 |

**Differential scanning calorimetry (DSC) method one of the present disclosure, with the test parameters shown in Table 5.**

**Table 5 Test parameters of DSC**

| Test parameters of DSC | |
|---|---|
| Instrument model | Mettler Toledo DSC1 |
| Method | Linear temperature increase |
| Sample pan | High pressure crucible |
| Temperature range | 40-350 °C |
| Scan rate (°C/min) | 10 |
| Protective gas | Nitrogen |

**Differential scanning calorimetry (DSC) method two of the present disclosure, with the test parameters shown in Table 6.**

**Table 6 Test parameters of DSC**

| Test parameters of DSC | |
|---|---|
| Instrument model | TA-DSC 250 |
| Method | Linear temperature increase |
| Sample pan | Aluminum crucible |
| Temperature range | 25-300 °C |
| Scan rate (°C/min) | 10 |
| Protective gas | Nitrogen |

**Thermogravimetric analysis (TGA) method one of the present disclosure, with the test parameters shown in Table 7.**

**Table 7 Test parameters of TGA**

| Test parameters of TGA | |
|---|---|
| Instrument model | TA Discovery TGA 550 |
| Method | Linear temperature increase |
| Sample pan | Aluminum pan, uncovered |
| Temperature range | Room temperature to 500 °C |
| Scan rate (°C/min) | 10 |
| Protective gas | Nitrogen |

### BRIEF DESCRIPTION OF DRAWINGS

Fig. 1: XRPD pattern of the crystal form A of the compound of formula (I) using Cu-Kα radiation;
Fig. 2: DSC curve of the crystal form A of the compound of formula (I);
Fig. 3 TGA curve of the crystal form A of the compound of formula (I);
Fig. 4: Ellipsoid diagram of the three-dimensional structure of the single crystal X-ray diffraction (SC-XRD) of the compound of formula (I);
Fig. 5: XRPD pattern of the crystal form B of the compound of formula (I) using Cu-Kα radiation;
Fig. 6: DSC curve of the crystal form B of the compound of formula (I).

### DETAILED DESCRIPTION

In order to better understand the content of the present disclosure, the present disclosure is further illustrated below in conjunction with specific examples, but the specific examples are not intended to limit the content of the present disclosure.

### Example 1

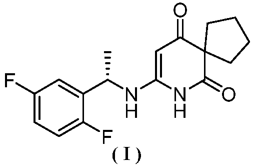

### Synthetic route:

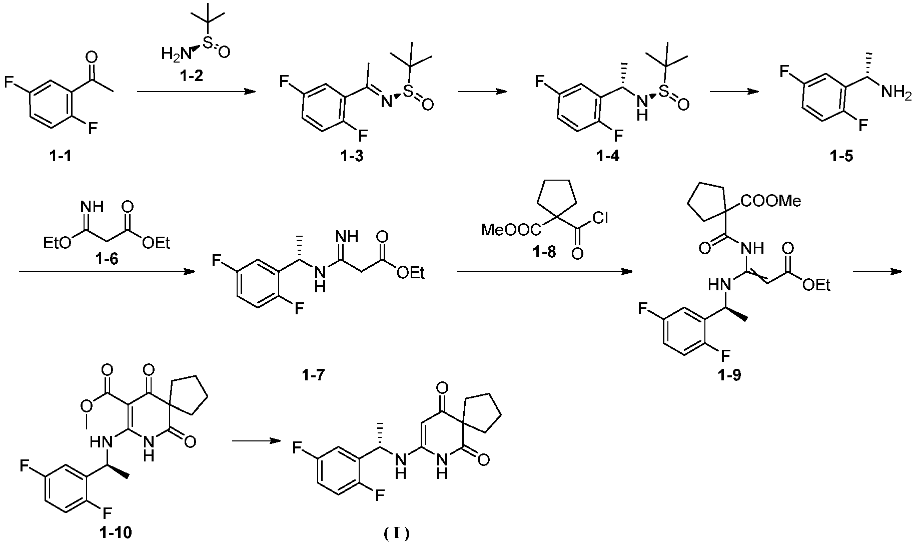

Step A: 1-2 (4.66 g, 38.43 mmol, 1.2 eq) and tetraethyl titanate (21.92 g, 96.07 mmol, 19.92 mL, 3 eq) were added to a solution of 1-1 (5 g, 32.02 mmol, 4.07 mL, 1 eq) in THF (50 mL) at 20 °C. The reaction solution was stirred at 60 °C for 16 hours. Ethyl acetate (100 mL) was added to the reaction solution. The mixture was cooled to 0 °C, and then water (20 mL) was slowly added. The mixture was stirred for 0.5 hours and filtered. The filtrate was washed with saturated brine (50 mL×3), dried over anhydrous sodium sulfate, filtered and then concentrated to give compound 1-3.

Step B: L-selectride (1 M, 41.65 mL, 1.2 eq) was slowly added dropwise to a solution of 1-3 (9 g, 34.71 mmol, 1 eq) in THF (100 mL) at -78 °C under nitrogen. The reaction solution was stirred at -78 °C for 2 hours and then slowly added to a saturated ammonium chloride aqueous solution (100 mL). The mixture was extracted with EA (100 mL × 2). The combined organic phase was washed with saturated brine (100 mL × 3), dried over anhydrous sodium sulfate, filtered and then concentrated. The residue was separated by column chromatography (PE:EtOAc = 5:1-3:1) to give compound 1-4.

Step C: HCl/MeOH (200 mmol, 50 mL, 7.92 eq) was added to a solution of 1-4 (6.6 g) in MeOH (50 mL) at 20 °C. The reaction solution was stirred for 16 hours and then concentrated to give a hydrochloride of compound 1-5.

Step D: 1-6 (1.51 g, 7.74 mmol, 1.5 eq, HCl) and DIEA(4.00 g, 30.96 mmol, 5.40 mL, 6 eq) were added to a solution of the hydrochloride of 1-5 (1 g) in EtOH (10 mL) at 20 °C. The reaction solution was stirred at 20 °C for 16 hours and concentrated to give compound 1-7.

Step E: TEA (2.45 g, 24.24 mmol, 3.37 mL, 3 eq) was added to a solution of 1-7 (1.54 g, 8.08 mmol, 1 eq) in DCM (15 mL) at -20 °C under nitrogen. Then, a solution of 1-8 (1.39 g, 5.14 mmol, 6.37e-1 eq) in DCM (15 mL) was added. The reaction solution was stirred at 20 °C for 16 hours, and then concentrated. The residue was diluted with added EA (30 mL), then washed with saturated brine (30 mL), dried over anhydrous sodium sulfate, filtered and then concentrated. The residue was separated by column chromatography (PE:EtOAc = 10:1-5:1) to give compound 1-9.

Step F: Sodium methoxide (1 M, 6.43 ml, 1 eq) was added to a solution of 1-9 (0.648 g, 1.53 mmol, 1 eq) in MeOH (7.6 mL) under nitrogen. The reaction solution was stirred at 20 °C for 16 hours. 1 M dilute hydrochloric acid was added to the reaction solution to adjust the pH to about 5, and then the mixture was extracted with added EA (20 mL). The organic phase was washed with saturated brine (20 mL), dried over anhydrous sodium sulfate, filtered and then concentrated to give compound 1-10.

Step G: Hydrochloric acid (4 M, 4 mL, 17.40 eq) was added to a solution of 1-10 (348 mg, 919.74 µmol, 1 eq) in 1,4-dioxane (4 mL). The reaction solution was stirred at 50 °C for 16 hours. EA (30 mL) was added to the reaction solution, and then a 1 M sodium hydroxide aqueous solution was added to adjust the pH to about 8. The layers were separated. Then, the organic phase was washed with saturated brine (50 mL × 2), dried over anhydrous sodium sulfate, filtered and then concentrated. MeOH (10 mL) was added to the residue, and the mixture was stirred for 20 minutes and filtered. The filter cake was dried under high vacuum to give the compound of formula (I). After XPRD testing, the obtained compound of formula (I) was the crystal form A of the compound of formula (I). ¹H NMR (DMSO-*d₆*, 400 MHz): δ ppm 9.60 - 9.47 (m, 1H), 7.34 - 7.26 (m, 2H), 7.25 - 7.16 (m, 1H), 6.96 (br d, *J=* 6.0 Hz, 1H), 4.82 - 4.71 (m, 1H), 4.44 - 4.34 (m, 1H), 1.93 - 1.81 (m, 4H), 1.70 (br s, 4H), 1.48 (br d, *J =* 6.4 Hz, 3H); LCMS(ESI) m/z: 321.2(M+1).

### Example 2 Preparation of the crystal form A of the compound of formula (I)

The compound of formula (I) (80 mg, 249.74 µmol) was added to ethanol (3 mL), and the mixture was stirred at 80 °C for 16 hours. The mixture was cooled to room temperature, and then filtered. The solid was vacuum dried to give the crystal form A of the compound of formula (I). ¹H NMR (DMSO-*d₆*, 400 MHz): δ ppm 9.60 - 9.47 (m, 1H), 7.34 - 7.26 (m, 2H), 7.25 - 7.16 (m, 1H), 6.96 (br d, *J =* 6.0 Hz, 1H), 4.82 - 4.71 (m, 1H), 4.44 - 4.34 (m, 1H), 1.93 - 1.81 (m, 4H), 1.70 (br s, 4H), 1.48 (br d, *J =* 6.4 Hz, 3H); LCMS(ESI) m/z: 321.2(M+1). The XRPD, DSC and TGA of the crystal form A of the compound of formula (I) were all tested using the corresponding method one, and the test result pattern and curves are shown in Figs. 1, 2 and 3, respectively.

The above solvent ethanol was replaced with methanol, dichloromethane, ethyl acetate, or tetrahydrofuran, and the crystal form A of the compound of formula (I) was obtained in each case at room temperature or under a heating temperature. The assay results are shown in Table 8.

**Table 8 Assay of crystal forms obtained by stirring in different solvents**

| Sample No. | Solvent (temperature) | Assay result |
|---|---|---|
| 1 | Methanol, 60 °C | Crystal form A |
| 2 | Dichloromethane, room temperature | Crystal form A |
| 3 | Ethyl acetate, room temperature | Crystal form A |
| 4 | Tetrahydrofuran, room temperature | Crystal form A |

### Example 3 Preparation of the crystal form B of the compound of formula (I)

About 60 mg of the crystal form A was weighed and heated from room temperature (20-25 °C) to 250 °C at a rate of 10 °C/min using TGA, and then naturally cooled (the cooling rate was not controlled during the cooling process) to room temperature to give crystal form B of the compound of formula (I). ¹H NMR (DMSO-*d₆*, 400 MHz): δ ppm 9.57 (s, 1H), 7.32 - 7.27 (m, 2H), 7.22 - 7.15 (m, 1H), 6.96 (br d, *J =* 6.4 Hz, 1H), 4.78 - 4.72 (m, 1H), 4.38 (s, 1H), 1.86 - 1.82 (m, 4H), 1.70 - 1.67 (m, 4H), 1.47 (br d, *J =* 6.8 Hz, 3H). The XRPD and DSC of the crystal form B of the compound of formula (I) were both tested using the corresponding method two, and the test result pattern and curve are shown in Figs. 5 and 6, respectively.

### Example 4: Single crystal X-ray diffraction analysis of the compound of formula (I)

### 1. Instrument parameters and data collection

Manufacturer: Bruker Corporation;
Instrument model: Bruker D8 VENTURE;
X-ray source: high-intensity micro focus rotating anode light source was used;
Cu rotating target: λ= 1.5418 4 Å;
Power: 2.5 kW;
Tube voltage: 50 kV;
Tube current: 50 mA;
Goniometer: four-axis (Kappa, ω, 2θ, ϕ) goniometer;
Detector: large-area photon II detector, with an effective area of 14 cm × 10 cm of the detector;
Distance from detector to sample: d = 45 mm.

### 2. Crystal cultivation

14.3 mg of sample was weighed and added to MeOH (2 mL). The sample was insoluble. Then, DMSO (1 mL) was added dropwise, and the mixture was stirred until completely dissolved. The dissolved sample was placed in a 4 mL semi-sealed sample vial and evaporated slowly at room temperature. After 7 days, a colorless block crystal was obtained and sent for single crystal testing.

### 3. Table of crystal data

**Table 9 Single crystal structure data of the compound of formula (I)**

| | | |
|---|---|---|
| Molecular formula | | C17H18F2N2O2 |
| Molecular mass | | 320.33 |
| Crystal system | | Orthorhombic crystal system |
| Space group | | *P*2₁2₁2₁ |
| Wavelength (Å) | | 1.54178 |
| Unit cell parameters | a/Å | 10.4611(2) |
| | b/Å | 11.8741(3) |
| | c/Å | 12.3305(3) |
| | α/° | 90 |
| | β/° | 90 |
| | γ/° | 90 |
| Unit cell volume (Å³) | | 1531.65(6) |

### 4. Conclusion

The single crystal data showed that the single crystal was the compound of formula (I). The ellipsoid diagram of the three-dimensional structure of the single crystal X-ray diffraction (SC-XRD) of the compound of formula (I) is shown in Fig. 4. The single crystal structure data and parameters of the compound of formula (I) are shown in Table 9.

### Biological assay data:

### Assay example 1: Assay of inhibitory effect on cardiac myosin ATPase activity

### Assay reagents:

Cardiac tropomyosin/troponin complex (Cytoskeleton, Cat. # TT05)
Cardiac myosin S1 (Cytoskeleton, Cat. # MYS03)
Cardiac actin (Cytoskeleton, Cat. # AD99-A)
ATPase assay biochem kit (Cytoskeleton, Cat. # BK051)

### Assay steps:

1) Preparation of compound
   a) The compound was serially diluted 4-fold with DMSO in Echo to 8 concentrations, and 200 nL of each concentration of the compound was transferred to a 96-well plate (Corning-3696), respectively.
   b) The plate was centrifuged at 1000 rpm for 15 seconds, and sealed for later use.
2) Preparation of F-actin
   a) A buffer of 5 mM Pipes-KOH pH 7.0, 500 µM ATP, and 500 µM dithiothreitol was prepared, and 2.5 mL of the buffer was added to dissolve 1 mg of F-actin to a protein concentration of 0.4 mg/mL.
   b) The mixture was placed at room temperature for 10 minutes to fully dissolve the protein.
   c) 2.0 mM MgCl₂ and 2.0 mM EGTA were added, and the mixture was placed at room temperature for 20 minutes to allow protein polymers to form.
3) Preparation of thin filament
   a) 200 µL of ice water was added to dissolve 1 mg of cardiac tropomyosin/troponin complex to a protein concentration of 5 mg/mL.
   b) 1000 µL of the F-actin prepared in step 1 was added and mixed well.
   c) The mixture was placed at room temperature for 20 minutes.
   d) The mixture was centrifuged at 87K × g at 4 °C for 1.5 hours.
   e) PM12 buffer (12 mM Pipes-KOH, pH 7.0, 2 mM MgCl₂) was prepared, and 1200 µL of the buffer was added to resuspend the protein.
4) Preparation of reaction solution and start of the assay
   a) 250 µL of ice-cold PM12 buffer was added to 250 µg of S1 myosin to a protein concentration of 1 mg/mL.
   b) The reagents were successively added in the following order and mixed to give a reaction mixture:
      400 µL of PM12,
      400 µL of 5× MSEG (from the ATPase assay biochem kit),
      1200 µL of actin/cardiac tropomyosin/troponin complex,
      40 µL of myosin S1,
      40 µL of 100x PNP (from the ATPase assay biochem kit),
      10.4 µL of 100 mM ATP.
   c) 10 µL of 440 µM CaCl₂ solution was added to the 96-well plate, and the plate was placed in a 37 °C incubator to pre-warm.
   d) 100 µL of the reaction mixture was added to the 96-well plate, and the plate was centrifuged at 1000 rpm for 10 seconds.
   e) The plate was read continuously for 10 min with a 30 seconds interval on a SpectraMax 340PC, with an instrument temperature of 37 °C and a wavelength of 360 nm.

### Data analysis:

The data were analyzed using Prism, and the assay result is shown in Table 10.

**Table 10 Assay result of IC₅₀ value of the inhibitory effect of the compound of the present disclosure on cardiac myosin ATPase activity**

| **Compound** | **IC₅₀ (µM)** |
|---|---|
| Compound of formula (I) | 0.22 |

**Conclusion:** The compound of the present disclosure has good inhibitory activity on cardiac myosin ATPase.

### Assay example 2: Pharmacokinetic evaluation in rats

### Purpose of the assay:

Pharmacokinetic parameters of the compound of the present disclosure in rats were assayed.

### Assay scheme:

1) Assay drug: The compound of the present disclosure;
2) Assay animals: Four male SD rats aged 7-9 weeks were randomly divided into two groups, with two rats in each group;
3) Drug preparation: An appropriate amount of the drug was weighed and dissolved in a mixed solvent of DMAC:PEG-400:30% 2-HP-0-CD = 5:25:70 to prepare 0.2 mg/mL;

### Assay procedure:

Animals in group 1 were administered the drug at a dosage of 0.2 mg/kg and a concentration of 0.2 mg/mL by a single injection via the tail vein. Animals in group 2 were administered the compound at a dosage of 1 mg/kg and a concentration of 0.2 mg/mL via oral gavage. Plasma samples were collected from the animals at 0.0833 (tail vein injection group only), 0.25, 0.5, 1, 2, 4, 6, 8, and 24 hours after administration.

### Data analysis:

The drug concentration in a plasma sample was determined using the LC-MS/MS method. The resulting pharmacokinetic assay results of the assay drug are shown in Table 11.

**Table 11 Pharmacokinetic assay results of the compound of the present disclosure**

| | | | | | | |
|---|---|---|---|---|---|---|
| **Compound of formula (I)** | Tail vein injection group | Clearance rate | Initial concentration | Volume of distribution | Half-life | Area under the curve |
| | | Cl (mL/Kg/min) | C₀ (nM) | Vd (L/Kg) | T_{1/2} (h) | AUC (nM•h) |
| | | 6.0 | 1460 | 2.7 | 6.37 | 1294 |
| | Oral gavage administration group | Maximum concentration | Time to maximum concentration | Area under the curve | Bioavailability | -- |
| | | Cₘₐₓ(nM) | T_{1/2}(h) | AUC (nM•h) | F (%) | -- |
| | | 1307 | 4.83 | 7929 | 123 | -- |

| | | | | | | |
|---|---|---|---|---|---|---|
| -- indicates absence. | | | | | | |

**Conclusion:** The compound of the present disclosure has good pharmacokinetic properties in rats.

## Claims

1. A crystal form A of a compound of formula (I), which is **characterized by** X-ray powder diffraction pattern (XRPD) with characteristic diffraction peaks at 2θ angles of 18.283±0.200°, 19.662±0.200°, and 22.420±0.200°;

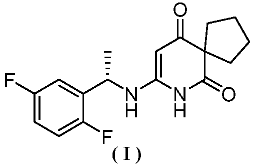

2. The crystal form A according to claim 1, which is **characterized by** X-ray powder diffraction pattern (XRPD) with characteristic diffraction peaks at 2θ angles of 11.143±0.200°, 18.283±0.200°, 19.662±0.200°, 22.420±0.200°, 26.259±0.200°, and 28.056±0.200°.

3. The crystal form A according to claim 2, which is **characterized by** X-ray powder diffraction pattern with characteristic diffraction peaks at 2θ angles of 11.143±0.200°, 18.283±0.200°, 19.662±0.200°, 22.420±0.200°, 26.259±0.200°, 27.261±0.200°, 28.056±0.200°, and 30.256±0.200°.

4. The crystal form A according to claim 3, which is **characterized by** X-ray powder diffraction pattern with characteristic diffraction peaks at 2θ angles of 11.143±0.200°, 16.522±0.200°, 17.053±0.200°, 18.283±0.200°, 19.662±0.200°, 22.420±0.200°, 23.909±0.200°, 26.259±0.200°, 27.261±0.200°, 28.056±0.200°, 30.256±0.200°, and 33.761±0.200°.

5. The crystal form A according to claim 1, which is **characterized by** X-ray powder diffraction pattern comprising at least 5, 6, 7 or 8 characteristic diffraction peaks represented by 2θ angles selected from the following: 11.143±0.200°, 18.283±0.200°, 19.662±0.200°, 22.420±0.200°, 26.259±0.200°, 27.261±0.200°, 28.056±0.200°, and 30.256±0.200°.

6. The crystal form A according to claim 5, which is **characterized by** X-ray powder diffraction pattern with characteristic diffraction peaks at 2θ angles of 11.143°, 13.260°, 14.860°, 16.163°, 16.522°, 17.053°, 17.537°, 18.283°, 19.662°, 22.420°, 23.568°, 23.909°, 26.259°, 26.726°, 27.261°, 28.056°, 29.008°, 30.256°, 31.219°, 31.646°, 32.037°, 32.438°, 32.807°, 33.761°, 34.534°, 35.404°, 36.856°, 37.813°, and 39.456°.

7. The crystal form A according to claim 1, which has an XRPD pattern substantially as shown in Fig. 1.

8. The crystal form A according to any one of claims 1 to 7, which has a differential scanning calorimetry (DSC) curve with peak values of the endothermic peaks at 249.53±3 °C and 306.12±3 °C.

9. The crystal form A according to claim 1, which has a DSC curve substantially as shown in Fig. 2.

10. The crystal form A according to any one of claims 1 to 7, which has a thermogravimetric analysis (TGA) curve with a weight loss of up to 0.075% at 200±3 °C, and a weight loss of up to 0.164% at 250±3 °C.

11. The crystal form A according to claim 1, which has a TGA curve substantially as shown in Fig. 3.

12. A method of preparing the crystal form A of the compound of formula (I) according to claim 1, comprising:
1) adding the compound of formula (I) into an alcohol solvent, ethyl acetate, tert-butyl methyl ether, tetrahydrofuran, or dichloromethane;
2) stirring at 15 to 100 °C for 1 to 168 hours;
3) filtering, collecting the filter cake, and vacuum drying the filter cake at 30 to 45 °C for 0.5 to 2 hours, alternatively vacuum drying the filter cake at 35 °C for 1 hour; wherein the compound of formula (I) is

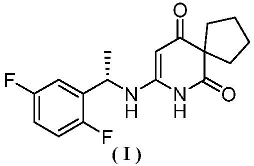

13. A pharmaceutical composition, comprising a therapeutically effective amount of the compound of formula (I) or the crystal form A of the compound of formula (I) according to any one of claims 1 to 11, and a pharmaceutically acceptable carrier.

14. Use of the compound of formula (I) or the crystal form A of the compound of formula (I) according to any one of claims 1 to 11, or the pharmaceutical composition according to claim 13 in the manufacture of a medicament as a cardiac myosin inhibitor Use of the pharmaceutical composition according to claim 13 in the manufacture of a medicament as a cardiac myosin inhibitor.

15. Use of the compound of formula (I) or the crystal form A of the compound of formula (I) according to any one of claims 1 to 11, or the pharmaceutical composition according to claim 13 in the manufacture of a medicament for treating heart failure and hypertrophic cardiomyopathy.
